(19) Europäisches Patentamt
European Patent Office
Office européen des brevets

(11) Publication number: **0 202 894 B1**

(12) **EUROPEAN PATENT SPECIFICATION**

(45) Date of publication of patent specification: **19.02.92** (51) Int. Cl.[5]: **G01N 33/68**, //G01N33/58

(21) Application number: **86303763.6**

(22) Date of filing: **16.05.86**

# (54) A method for detecting amino acid derivatives.

(30) Priority: **17.05.85 JP 105400/85**

(43) Date of publication of application:
**26.11.86 Bulletin 86/48**

(45) Publication of the grant of the patent:
**19.02.92 Bulletin 92/08**

(84) Designated Contracting States:
**DE FR GB**

(56) References cited:
**US-A- 3 645 689**

**ACTA CHEMICA SCANDINAVICA, vol. 10, 1956, pages 761-768, SE; P. EDMAN: "On the mechanism of the phenyl isothiocyanate degradation of peptides"**

**AUST. J. CHEM., vol. 28, 1975, pages 2289-2298, Melbourne, AUS; C.J. BURRELL et al.: "The use of rho-Iodophenyl[125I] Isothiocyanate for determination of N-terminal amino acids in nanomole quantities of protein"**

**TRAC: TRENDS IN ANALYTICAL CHEMISTRY, vol. 2, no. 12, December 1983, pages 267-269, Cambridge, GB; D.G. KLAPPER: "Trends in automated protein sequence analysis"**

(73) Proprietor: **SEIKO INSTRUMENTS INC.**
**31-1, Kameido 6-chome Koto-ku**
**Tokyo 136(JP)**

(72) Inventor: **Tsugita, Akira**
**7-8, Tsukumodai 5-chome**
**Suita-shi Osaka(JP)**
Inventor: **Arai, Isamu**
**1704, Kumakawa**
**Fussa-shi Tokyo(JP)**
Inventor: **Ataka, Tatsuaki Seiko Instr.&Electronics Ltd.**
**31-1, Kameido 6-chome**
**Koto-ku Tokyo(JP)**

(74) Representative: **Miller, Joseph et al**
**J. MILLER & CO. Lincoln House 296-302 High Holborn**
**London WC1V 7JH(GB)**

EP 0 202 894 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to a method for detecting amino acids, and it relates more especially to a method for detecting amino acid derivatives, for example for application to N-terminal group determination of a protein.

It is known to detect amino acids using the Edman method, P. Edman, Acta. Chem. Scand. 10 761 (1956). The Edman method is an N-terminal group determination method. In the final step of the PTC method according to the Edman method, it has been usual practice to treat thiazolinone derivatives with an acid to form phenylthiohydantoin (PTH) derivatives, and to determine these derivatives absorptiometrically. Although this known method for absorptiometrically detecting phenylthiohydantoin derivatives is a simple and convenient detection means, it cannot fully cope with a recent trend towards more highly sensitive analysis of a protein with a smaller amount of specimen.

For high sensitivity detection of amino acids, methods $^{32}$S PITC labelling of $^{135}$1 PITC labelling are disclosed in (1) W.G. Lauer, Fundamental Techniques in Virology, Eds. K. Habel and N. P. Salgman, p. 379 (1969) Academic Press N. Y., and (2) C. J. Burrell, P. D. Cooper, J. M. Swann, Aust. J. Chem. 28 2289 (1975). In the disclosed methods, radio-active isotope derivatives take part in the main reaction in the Edman degradation method. However, if high radio-activity is used in order to obtain high sensitivity, radio-active disintegration increases which adversely affects yield in the Edman amino acid sequence determination, and also the contamination of reaction vessels occurs.

It is an aim of the present invention to provide a method for detecting amino acid derivatives in which the above mentioned problems are obviated or reduced.

Accordingly, this invention provides a method for detecting amino acid derivatives, which method is characterised by reacting 5-thiazolinone amino acid derivatives of the general formula (a) below, with an amino compound which is of the general formula (b) below and which is labelled with a radio-active iodine isotope or which is a fluorescent amino compound, to form phenylthiocarbamylamino acid derivatives of the general formula (c) below, and detecting the phenyl thiocarbamylamino acid derivatives

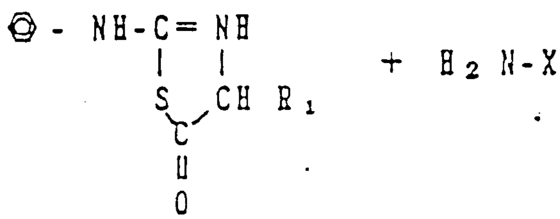

(a) (b)

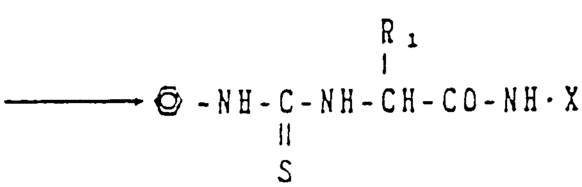

( I )

(c)

wherein $R_1$, $R_2$ and $R_3$ represent amino acid side chains and wherein X is a residue of a radio-active iodine isotope labelled or fluorescent compound.

Preferably, the 5-thiazolinone amino acid derivatives are obtained by reacting phenyl isothiocyanate of the general formula (d) below with a protein or a peptide of the general formula (e) below to form phenylthiocarbamylprotein of the general formula (f) below, and reacting the phenylthiocarbamylprotein with an acid in an anhydrous condition according to the formula [III] below to effect cyclization and scission:

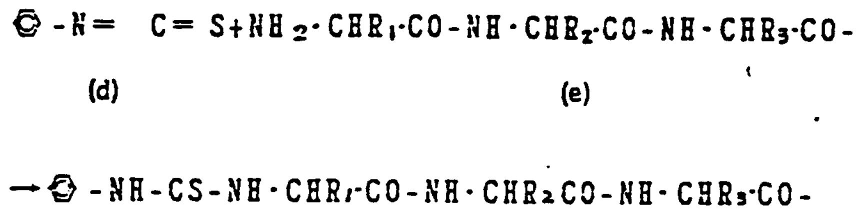

(d) (e)

(f) ( II )

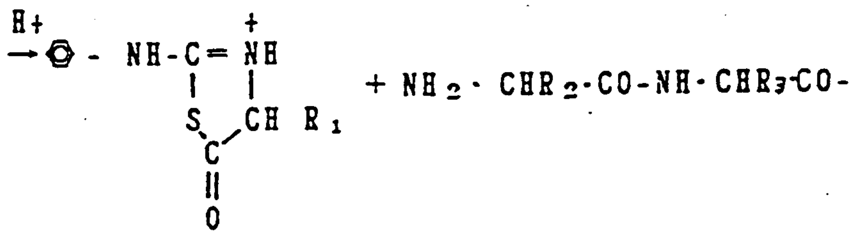

(a) ( III )

wherein $R_1$, $R_2$ and $R_3$ represent amino acid side chains.

The method of the invention may enable the amino acid derivatives to be detected with a high degree of sensitivity. Also, smaller amounts of specimens may be used as compared with the amounts of specimens used in known methods. Thus, the method of the invention is of considerable industrial importance and value.

The amino compound of the general formula (b) may be a radio-active iodine isotope labelled iodohistamine. Alternatively, the amino compound of the general formula (b) may be a fluorescent amino compound in the form of aminopyrene or aminofluorene.

Preferably the phenylthiocarbamylamino acid derivatives are detected by using a gamma ray detector or a fluorescence spectrophotometer.

The amino acid side chains for $R_1$, $R_2$ and $R_3$ are preferably hydrogen or hydrocarbon radicals.

The invention will now be further illustrated, merely by way of example, with reference to the accompanying drawings in which:-

Figure 1 is a process flow sheet showing the detection method of the invention;

Figure 2 is a process flow sheet showing a known method;

Figure 3 shows three high pressure liquid chromatography charts or graphs of a reaction mixture and a reaction product (obtained by the mixing ATZ-Leu (which abbreviations are hereinbelow explained) and iodohist-amine), the charts being such that:

Figure 3 (i) illustrates just after mixing;

Figure 3 (ii) illustrates after heating at 50 °C for 30 minutes, and

Figure 3 (iii) illustrates after heating at 50 °C for one hour a thiazolinone derivative of leucine and phenylthiocarbamyl derivative of leucine;

Figure 4 is a thin layer chromatogram of various phenylthiocarbamylamino acids; and

Figure 5 shows two high pressure liquid chromatography charts or graphs of a reaction mixture and a reaction product (obtained by the reaction between ATZ-Leu and aminofluorene).

In order to facilitate a full and complete understanding of the present invention, reference will also be made to the following Examples.

## EXAMPLE 1

This Example demonstates a basic detection method wherein 5-thiazolinone derivatives of amino acids (herein after abbreviated to ATZ) were reacted with a radioactive iodine isotope labelled iodohistamine to form phenylthiocarbamylamino acid derivatives, and these phenylthiocarbamylamino acid derivatives were

detected with a high level of sensitivity.

In the basic detection method a dipeptide (herein after abbreviated as Leu-Ala) consisting of leucine (herein after abbreviated as Leu) and alanine (herein after abbreviated as Ala) is used instead of a protein for simplicity. The basic detection method is described in the following order:

1. Phenylisothiocyanate (PITC)-Leu-Ala and ATZ-Leu
   - i) synthesis of PITC-Leu-Ala
   - ii) synthesis and purification of ATZ-Leu
2. Coupling reaction
3. Detection of phenylthiocarbamylamino acid derivatives.

1. PITC-Leu-Ala and ATZ-Leu

i) Synthesis of PITC-Leu-Ala

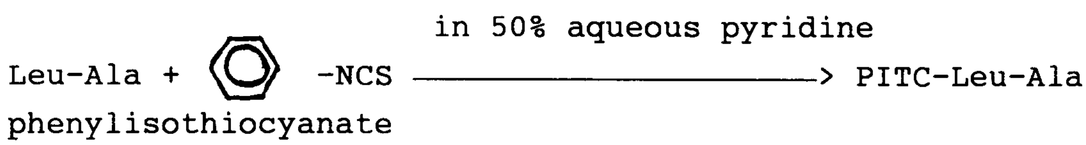

Leu-Ala + phenylisothiocyanate → PITC-Leu-Ala

Leu-Ala (202 mg) was dissolved in 50% aqueous pyridine, and the pH was adjusted to 8.6 by the addition of 2N sodium hydroxide (NaOH). Subsequently, phenylisothiocyanate was added thereto. The pH was kept at 8.6 by the addition of 2N sodium hydroxide because it was decreasing with the addition of the phenylisothiocyanate. After the variation in the pH had been decreased, the solution was heated at 40°C for one hour. After the reaction, the reaction solution was washed with benzene. After the benzene dissolved in the water phase had been purged with nitrogen ($N_2$) gas, the pH was adjusted to 2 by the addition of 1N hydrochloric acid (HCl), whereupon PITC-Leu-Ala was obtained in the form of a white precipitate. Yield: 270 mg, % yield: 80%, melting point: 148°C.

ii) Synthesis and purification of ATZ-Leu

TFA

PITC-Leu-Ala ———————— ATZ-Leu + Ala

The above PITC-Leu-Ala (100 mg) was dissolved in TFA (1 ml), and the solution was heated at 50°C for 5 minutes. After the reaction, the solution was completely evaporated to dryness. Butyl chloride was added to the residue to thoroughly dissolve the product in it, and the resulting solution was passed through a cellulose column (Ala was adsorbed on the column). The effluent was collected and evaporated to dryness. ATZ-Leu was obtained in the form of a white solid.

Yield: 70 mg, % yield: 95%

2. Coupling reaction

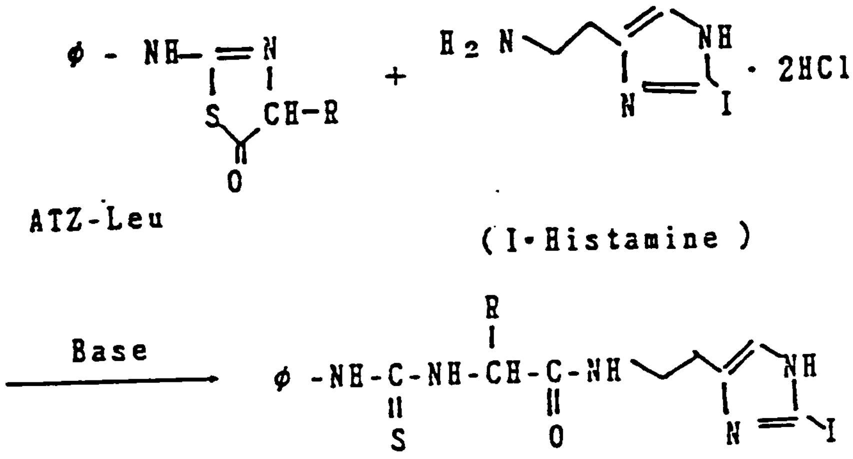


ATZ-Leu

(I•Histamine)

As the above reaction formula shows, the amino group of iodohistamine (herein after referred to as I. histamine) attacks the carbonyl group of ATZ amino acid to give a product. The reaction is the reverse of a reaction for cleaving the peptide bond between phenylisothiocyanate and the peptide. In the above reaction formula, $\phi$ and R are any known and suitable chemical parts, groups or radicals. R, for example, may be amino acid side chains, e.g. hydrogen or hydrocarbon radicals, and $\phi$ may be the phenyl radical.

ATZ-Leu (70 mg) obtained from PITC-Leu-Ala was dissolved in 30% pyridine/dimethylformamide (20 ml), to which was then added I. histamine 2HCl (200 mg). The resulting mixture was heated with agitation at 50°C for one hour. The reaction solution was evaporated to dryness and the residue was dried to solid form and extracted with 1N sodium bicarbonate and ethyl acetate. The ethyl acetate phase was dried and evaporated to dryness. The residue was re-crystallised from benzene to obtain a product in the form of a white crystal.

Yield: 120 mg, % yield: 83%.

There was good agreement between the result of elementary analysis of this product and the values calculated from its theoretical formula.

3. Detection of phenylthiocarbamylamino acid derivatives

Each of the reaction mixture and the reaction product was passed through a high pressure liquid chromatography column and the detection was made by ultra-violet fluorescence spectra (at 269 nm). The results are shown in Figure 3. Figure 3 (i) shows a high pressure liquid chromatography chart just after mixing. Figure 3 (ii) shows a high pressure liquid chromatography chart after heating at 50°C for thirty minutes. Figure 3 (iii) shows a high pressure liquid chromatography chart after heating at 50°C for one hour. As these Figures clearly show, the coupling reaction between the thiazolinone derivative of leucine and iodohistamine was completed by heating at 50°C for one hour, so that the thiazolinone derivative was detected in the form of a phenylthiocarbamylleucine derivative.

Conditions of high pressure liquid chromatography

SERVA Cat No. 4231
250 mm x $\phi$ 4.6 mm
SERVACHROM Packing
Si100 = polyol RP18
Particle size 5 microns

Solvent system:

| buffer solution | organic solvent |
|---|---|
| 0.015 M NaOAc (pH = 5 with AcOH) | $CH_3CH$-MeOH (4:1) |
| 1 | 5 |

effluent flow rate: 1.5 ml/min
detection: UV 269 nm
chart speed: 10 mm/min

EXAMPLE 2

This Example demonstrates how a method similar to that described in Example 1 was applied to various dipeptides and a pentapeptide, and the detection was performed by using thin layer chromatography and an X-ray film.

The dipeptides and heptapeptide used were as follows: Leu-Gly, Pro-Phe, Phe-Ala, Met-Leu, Ser-Phe, Ile-Ser, Gly-Glu, Ala-Ala, Val-Glu, Gln-Gly and Tyr-Gly-Gly-Phe-Leu.

A similar method to that described in Example 1 was followed. Thus, each of the above peptides was reacted with phenylisothiocyanate to form a phenylthiopeptide, which was converted into a thiazolinone derivative by cyclization and scission by treatment with trifluoroacetic acid. The thiazolinone derivative was reacted with iodohistamine partially labelled with a radio-active isotope to form a phenylthiocarbamylamino acid derivative. A variety of the formed phenylthiocarbamylamino acid derivatives were passed through a thin layer chromatograpic column and then detected as clear exposed spots on an X-ray film after exposure for day and night. In the thin layer chromatography, a solvent system comprising benzene, methanol and tertbutyl alcohol in a volume ratio of 8:1:1 was used in development.

The results are shown in Figure 4, wherein ordinates represent radio frequency (RF) values. As compared with the sensitivity of detection in the prior art, the level of sensitivity of the present invention was extremely high as follows:

| Detection method | Sensitivity of detection |
|---|---|
| Known method using phenylthiohydantoinamino acid detection<br>This invention | ~ 10 p mole<br>0.1 ~ 10 f mole |

In this Example, no radio-active substance is used in the main reaction. It is used only in the final step in the sequence determination method, so that the main reaction is quite the same as in the prior art. A radio-active substance is used in a special vessel in only the final modification reaction of a reaction product intermediate (ATZ) in each step and therefore the reaction itself does not differ at all from that in the prior art. Therefore, it is possible to perform the reaction without adversely affecting the reaction yield, while minimising radio-active contamination.

EXAMPLE 3

This Example demonstrates how 5-thiazolinone derivatives of amino acids were reacted with a fluorescent amino compound (9-aminofluorene) to form phenylthiocarbamylamino acid derivatives, and the products were detected with high sensitivity.

A similar procedure to that described in Example 1 was followed until the step of obtaining thiazolinone derivatives. The thiazolinone derivative of leucine (herein after abbreviated as ATZ-Leu) was used.

This reaction proceeded according to the following reaction formula to form a phenylthiocarbamylamino acid derivative.

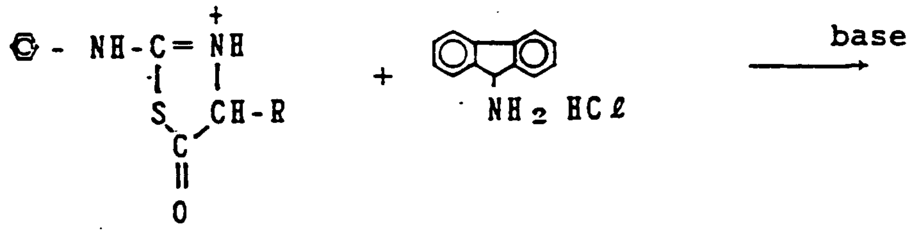


thiazolinone derivative 9-aminofluorene

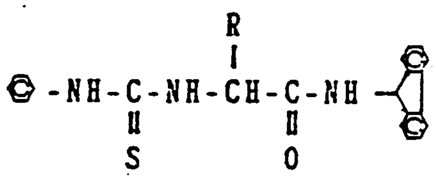

R : Leu

phenylthiocarbamylamino acid derivative.

In the above reaction formula, R is an amino acid side chain, e.g. a hydrogen or hydrocarbon radical.

The reaction was performed in the following way. To ATZ-Leu (70 mg, 0.21 mM) was added 9-aminofluorene (130 mg, 0.6 mM) dissolved in 30% pyridine-dimethylformamide, and the resulting mixture was reacted by heating at 50 °C for one hour.

After the reaction, the reaction solution was evaporated to dryness. Chloroform ($CHCl_3$) was added to the residue and the insoluble substance (9-aminofluorene HCl) was separated by filtration. The chloroform phase was washed with 0.1 N HCl and 0.11 N $NaHCO_3$, over $Na_2SO_4$ and concentrated to dryness. The residue was re-crystallised from benzene to obtain a product in the form of a white crystal.

Yield: 130 mg, % yield: 88%, melting point: 225 °C.

Each of the reaction mixture and the reaction product was passed through a high pressure liquid chromatography column, and the detection was performed by ultra-violet fluorescence spectra (at 269 nm). The results are shown in Figure 5 with Figure 5 (i) showing a high pressure liquid chromatography chart of the reaction mixture just after mixing, and Figure 5 (ii) shows a high pressure liquid chromatography chart after reacting by heating at 50 °C for one hour. As these Figures 5 (i) and 5 (ii) clearly show, a thiazolinone derivative (a) of leucine after the reaction was detected in the form of a phenylthiocarbamylleucine derivative (c). In addition, comparison between the sensitivity of detection in this invention and that in the prior art phenylthiohydantoinamino acid detection method revealed that the sensitivity of detection in this invention was extremely high as follows:

| Detection method | Sensitivity of detection |
|---|---|
| Known method using phenylthiohydantoinamino acid | ~ 10 p mole |
| This invention | 0.1 ~ 1 p mole |

In accordance with this invention, extremely high sensitivity of detection of the amino acid derivatives is shown below by comparison with the sensitivity of detection in the prior art method in which a phenylthiocarbamylamino acid derivative is used:

| Detection method | Sensitivity of detection |
|---|---|
| Known method using phenylthiohydantoinamino acid | 1 ~ 10 p mole |
| This invention: when iodine isotope labelling is used | 0.1 ~ 10 f mole |
| when a fluorescent amino compound is used | 0.1 ~ 1 p mole |

It will be appreciated from the above Figures and Examples, that this invention resides in reacting an

iodine labelled or a fluorescent amino compound with a thiazolinone derivative of an amino acid to form a phenylthiocarbamyl derivative and detecting it with a high degree of sensitivity. Although the above Examples only use iodohistamine or aminofluorene, it is to be understood that other iodine labelled or fluorescent amino compounds can be used, and that this invention is not to be limited to the particular chemicals or compounds used in the Examples.

In the Examples, the following abbreviations have the following meanings:

PTC = Phenylisothiocyanate
TFA = Trifluoroacetic acid
LEU-GLY = leucylglycine
PRO-PHE = prolylphenylalanine
PHE-ALA = phenylalanylalanine
MET-LEU = methionylleucine
SER-PHE = serylphenylalanine
ILE-SER = isoleucylserine
GLY-GLU = glycylglutamic acid
VAL-GLU = valylglutamic acid
GLN-GLY = glutaminylglycine
TYR-GLY-GLY-PHE-LEU = tyrosylglycylglycylphenylalanylleucine

## Claims

1. A method for detecting amino acid derivatives, which method is characterised by reacting 5-thiazolinone amino acid derivatives of the general formula (a) below, with an amino compound which is of the general formula (b) below and which is labelled with a radio-active iodine isotope or which is a fluorescent amino compound, to form phenylthiocarbamylamino acid derivatives of the general formula (c) below, and detecting the phenylthiocarbamylamino acid derivatives

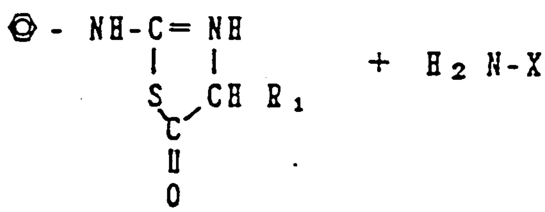

(a) (b)

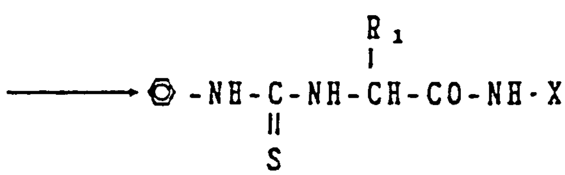

[ I ]

(c)

wherein $R_1$, $R_2$ and $R_3$ represent amino acid side chains and wherein X is a residue of a radio-active iodine isotope labelled or fluorescent compound.

2. A method according to claim 1, characterised in that the 5-thiazolinone amino acid derivatives are obtained by reacting phenyl isothiocyanate of the general formula (d) below with a protein or a peptide of the general formula (e) below to form phenylthiocarbamylprotein of the general formula (f) below, and reacting the phenylthiocarbamylprotein with an acid in an anhydrous condition according to the formula [III] below to effect cyclization and scission:

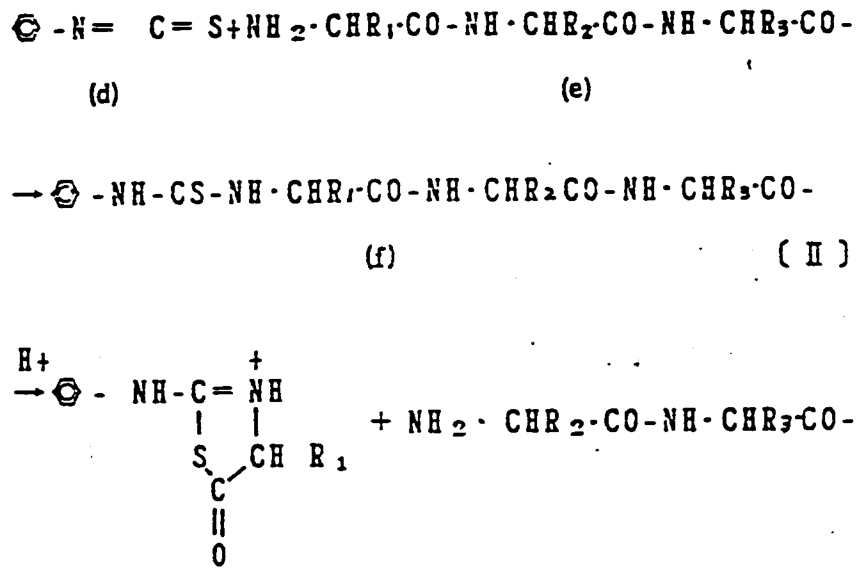

(d) (e)

(f) ( II )

(a) ( III )

wherein $R_1$, $R_2$ and $R_3$ represent amino acid side chains.

**3.** A method according to claim 1 or claim 2, characterised in that the amino compound of the general formula (b) is a radio-active iodine isotope labelled iodohistamine.

**4.** A method according to any one of the preceding claims, characterised in that the amino compound of the general formula (b) is a fluorescent amino compound in the form of aminopyrene or aminofluorene.

**5.** A method according to any one of the preceding claims, characterised in that the phenylthiocarbamylamino acid derivatives are detected by using a gamma ray detector or a fluorescence spectrophotometer.

**6.** A method according to any one of the preceding claims characterised in that the amino acid side chains for $R_1$, $R_2$ and $R_3$ are hydrogen or hydrocarbon radicals.

**Revendications**

**1.** Procédé de détection de dérivés d'acides aminés, caractérisé en ce qu'il consiste à faire réagir des dérivés d'acide 5-thiazolinone aminés de formule développée (a) ci-dessous, sur un composé aminé de formule développée (b) ci-dessous, qui est marqué par un isotope radioactif d'iode ou qui est un composé aminé fluorescent, pour former des dérivés d'acide phénylthiocarbamylaminés de formule développée (c) ci-dessous, et à détecter les dérivés d'acides phénylthiocarbamylaminés

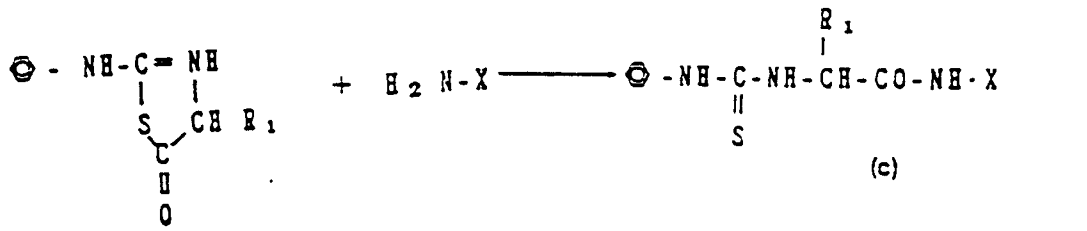

( I )

(c)

(a) (b)

dans lesquelles $R_1$ représente une chaîne latérale d'acide aminé et X est un résidu d'un composé

marqué par un isotope radioactif d'iode ou d'un composé fluorescent.

2. Procédé suivant la revendication 1, caractérisé en ce qu'il consiste à obtenir les dérivés d'acide 5-thiazolidone aminés en faisant réagir de l'isothiocyanate de phényle de formule développée (d) ci-dessous, sur une protéine ou sur un peptide de formule développée (e) ci-dessous, pour former une phénylthiocarbamylprotéine de formule développée (f) ci-dessous, et à faire réagir la phénylthiocarbamylprotéine sur un acide dans des conditions anhydre suivant l'équation [III] ci-dessous pour effectuer une cyclisation et une scission :

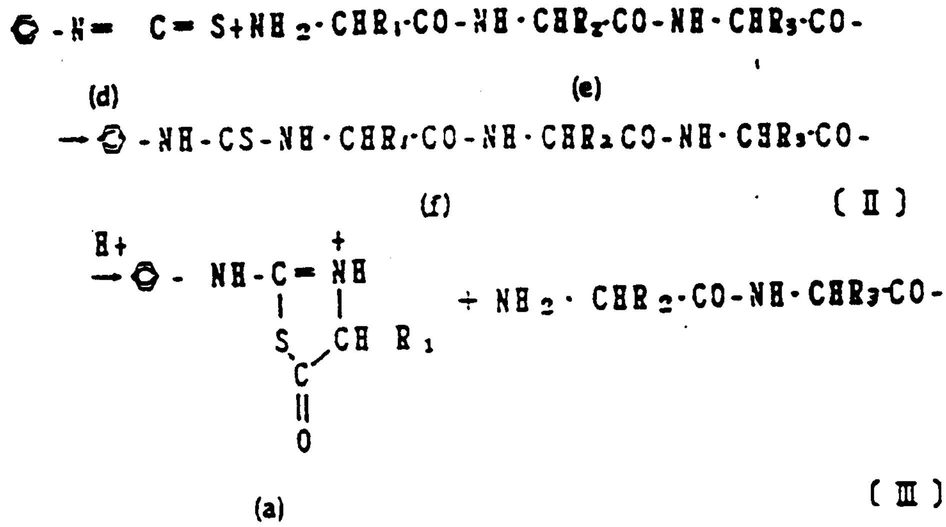

(d) (e) (f) ( II ) (a) ( III )

dans lesquelles $R_1$, $R_2$ et $R_3$ représentent des chaînes latérales d'acide aminé.

3. Procédé suivant la revendication 1 ou 2, caractérisé en ce que le composé aminé de formule développée (b) est de l'iodohistamine marquée par un isotope radioactif de l'iode.

4. Procédé suivant l'une quelconque des revendications précédentes, caractérisé en ce que le composé aminé de formule développée (b) est un composé aminé fluorescent sous la forme d'un aminopyrène ou d'un aminofluorène.

5. Procédé suivant l'une quelconque des revendications précédentes, caractérisé en ce qu'il consiste à détecter les dérivés d'acides phénylthiocarbamylaminés, en utilisant un détecteur de rayons gamma ou un spectrophotomètre à fluorescence.

6. Procédé suivant l'une quelconque des revendications précédentes, caractérisé en ce que les chaînes latérales d'acide aminé pour $R_1$, $R_2$ et $R_3$ sont de l'hydrogène ou des radicaux hydrocarbonés.

**Patentansprüche**

1. Verfahren zum Nachweis von Aminosäurederivaten, wobei das Verfahren **dadurch gekennzeichnet** ist, daß man 5-Thiazolinon-Aminosäurederivate der folgenden allgemeinen Formel (a) mit einer Aminoverbindung umsetzt, welche die folgende allgemeine Formel (b) aufweist und mit einem radioaktiven Jodisotop markiert ist oder eine fluoreszierende Aminoverbindung ist, um Phenylthiocarbamyl-Aminosäurederivateder folgenden allgemeinen Formel (c) herzustellen, und die Phenylthiocarbamyl-Aminosäurederivate nachweist

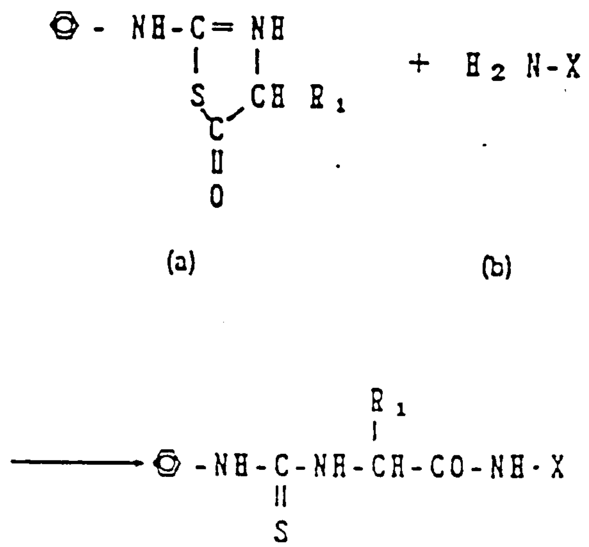

(a) (b)

(c) ( I )

worin $R_1$ Aminosäureseitenketten darstellen und worin X ein Rest einer mit einem radioaktiven Jodisotop markierten oder fluoreszierenden Verbindung ist.

2. Verfahren nach Anspruch 1, **dadurch gekennzeichnet,** daß die 5-Thiazolinon-Aminosäurederivate durch Reaktion eines Phenylisothiocyanats der folgenden allgemeinen Formel (d) mit einem Protein oder Peptid der folgenden allgemeinen Formel (e) unter Bildung von Phenylthiocarbamyl-Protein der folgenden allgemeinen Formel (f) und Reaktion des Phenylthiocarbamyl-Proteins mit einer Säure in wasserfreiem Zustand gemaß Formel (III), um Cyclisierung und Spaltung zu bewirken, erhalten werden:

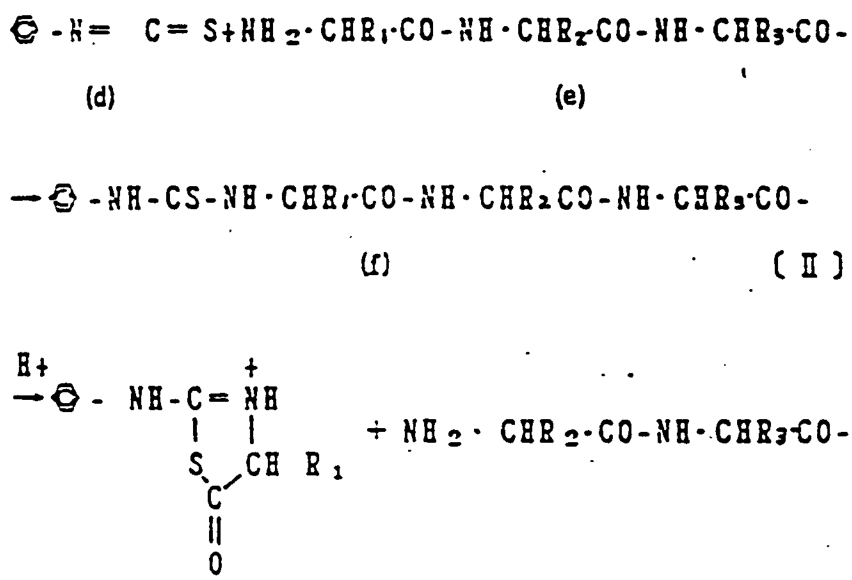

(d) (e)

(f) ( II )

(a) ( III )

worin $R_1$, $R_2$ und $R_3$ Aminosäureseitenketten darstellen.

3. Verfahren nach Anspruch 1 oder 2, **dadurch gekennzeichnet,** daß die Aminoverbindung der allgemeinen Formel (b) ein mit einem radioaktiven Jodisotop markiertes Jodhistamin ist.

4. Verfahren nach einem der vorhergehenden Ansprüche, **dadurch gekennzeichnet,** daß die Aminoverbindung der allgemeinen Formel (b) eine fluoreszierende Aminoverbindung in Form von Aminopyren oder Aminofluoren ist.

5. Verfahren nach der vorhergehenden Ansprüche, **dadurch gekennzeichnet,** daß die Phenylthiocarbamyl-Aminosäurederivate durch Verwendung eines Gammastrahlendetektors oder eines Fluoreszenzspektrometers nachgewiesen werden.

6. Verfahren nach der vorhergehenden Ansprüche, **dadurch gekennzeichnet,** daß die Aminoäureseitenketten für $R_1$, $R_2$ und $R_3$ Wasserstoff- oder Kohlenwasserstoffreste sind.

FIG.1

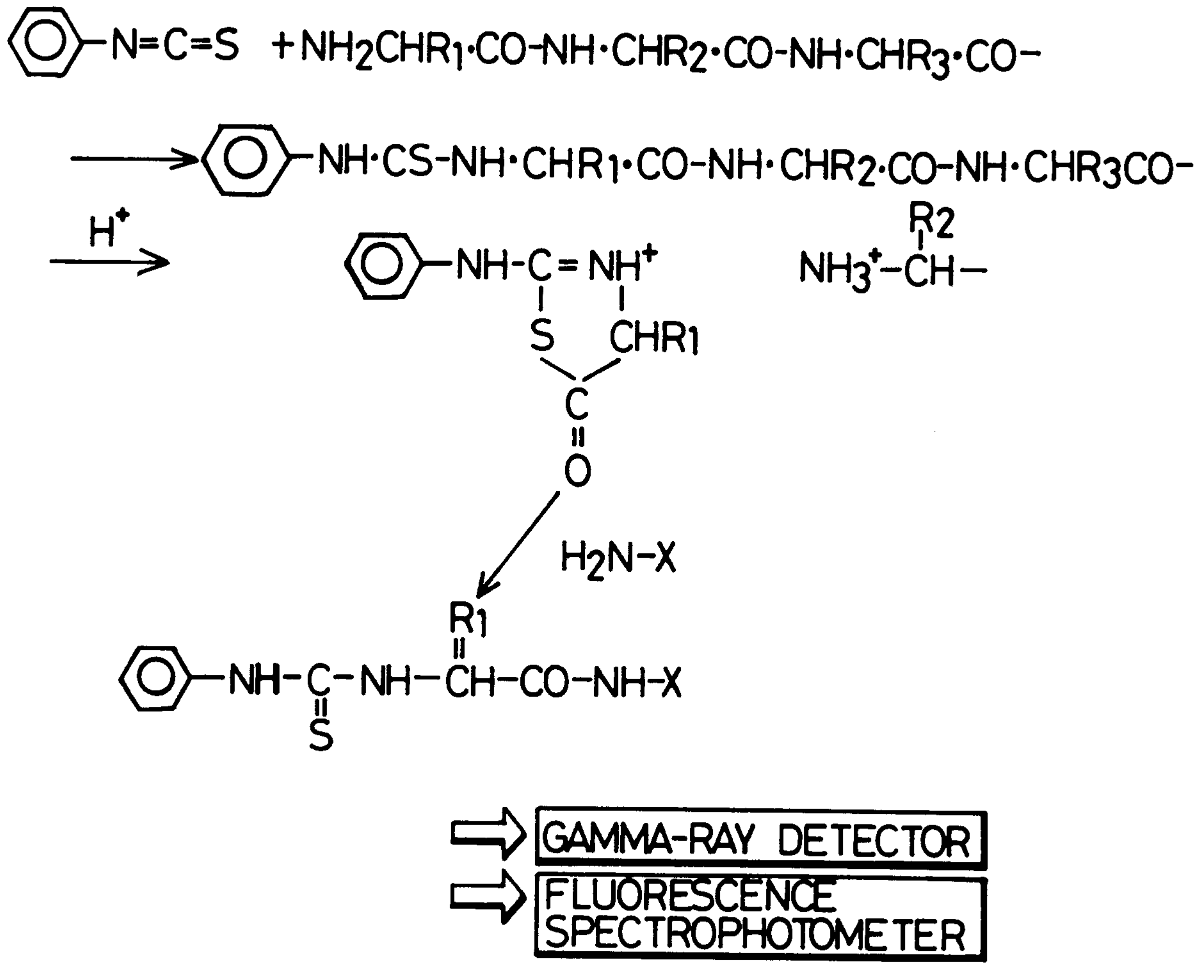
H⁺
H2N-X
GAMMA-RAY DETECTOR
FLUORESCENCE SPECTROPHOTOMETER

F I G. 2

PROCESS FLOWSHEET OF PRIOR ART METHOD

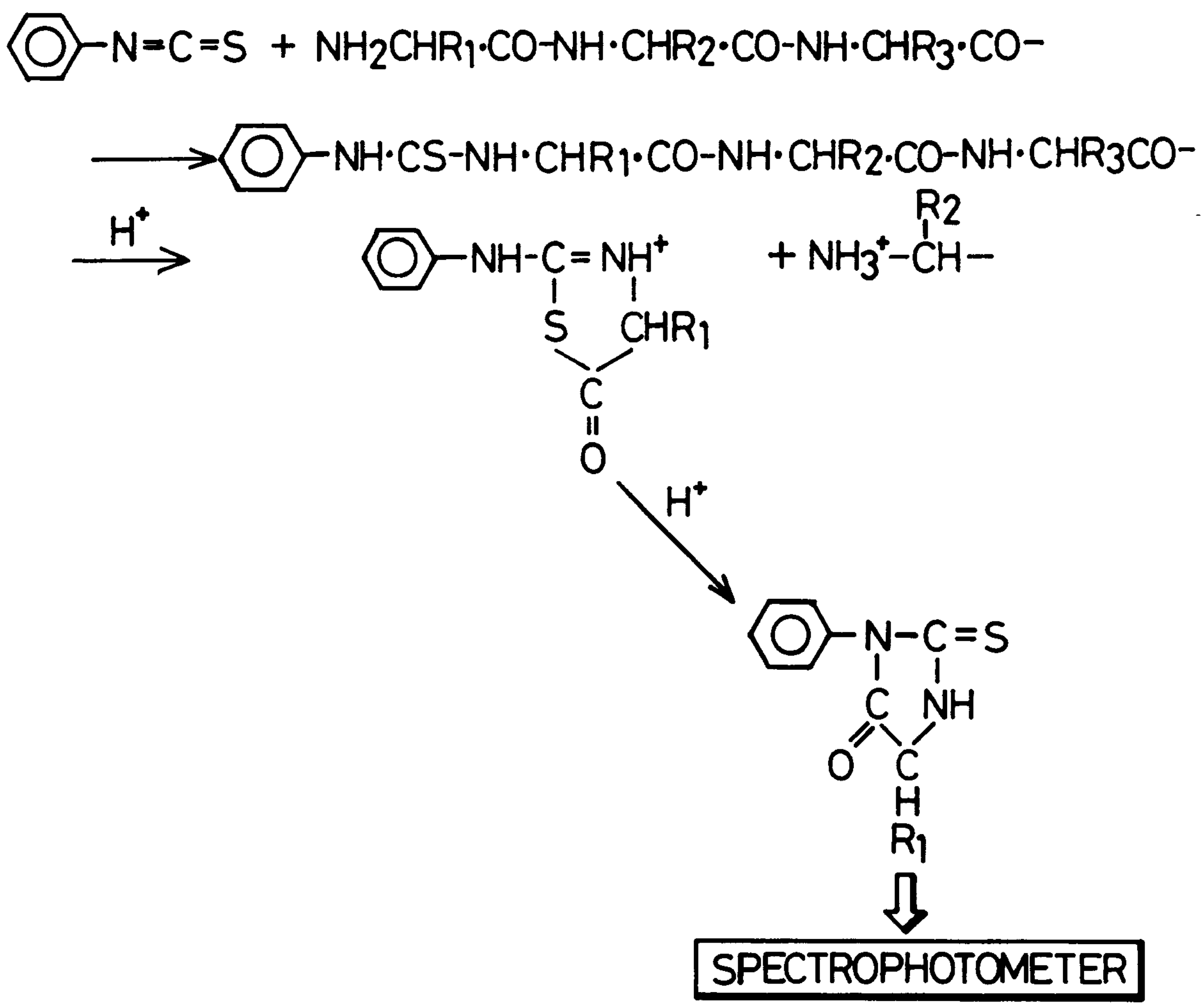

FIG. 3

(i)

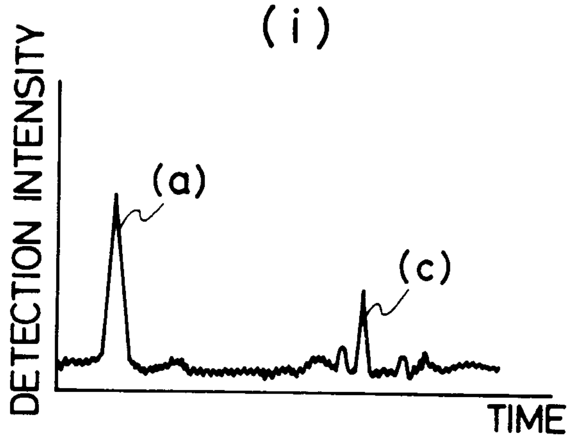
DETECTION INTENSITY
(a)
(c)
TIME

(ii)

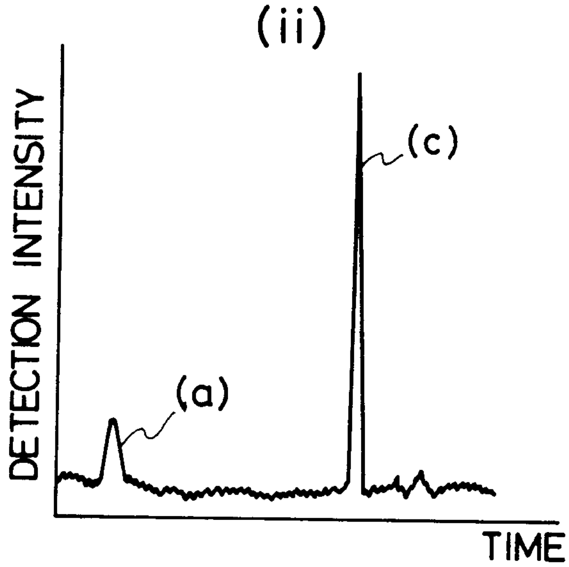
DETECTION INTENSITY
(c)
(a)
TIME

(iii)

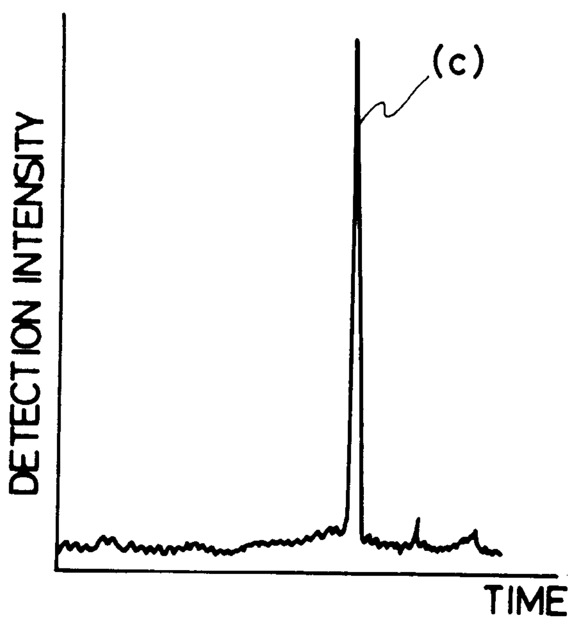
DETECTION INTENSITY
(c)
TIME

FIG.4

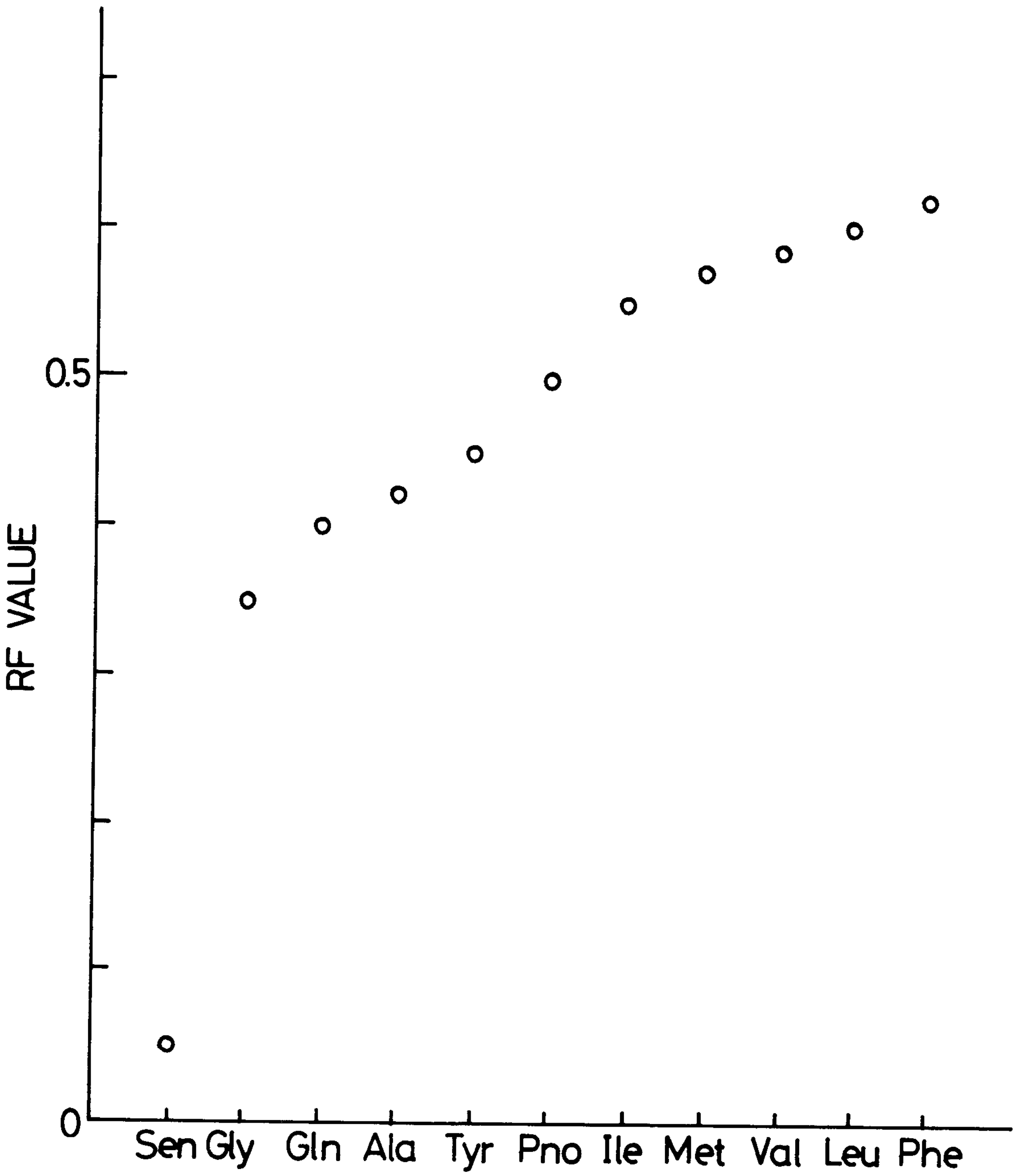

FIG. 5

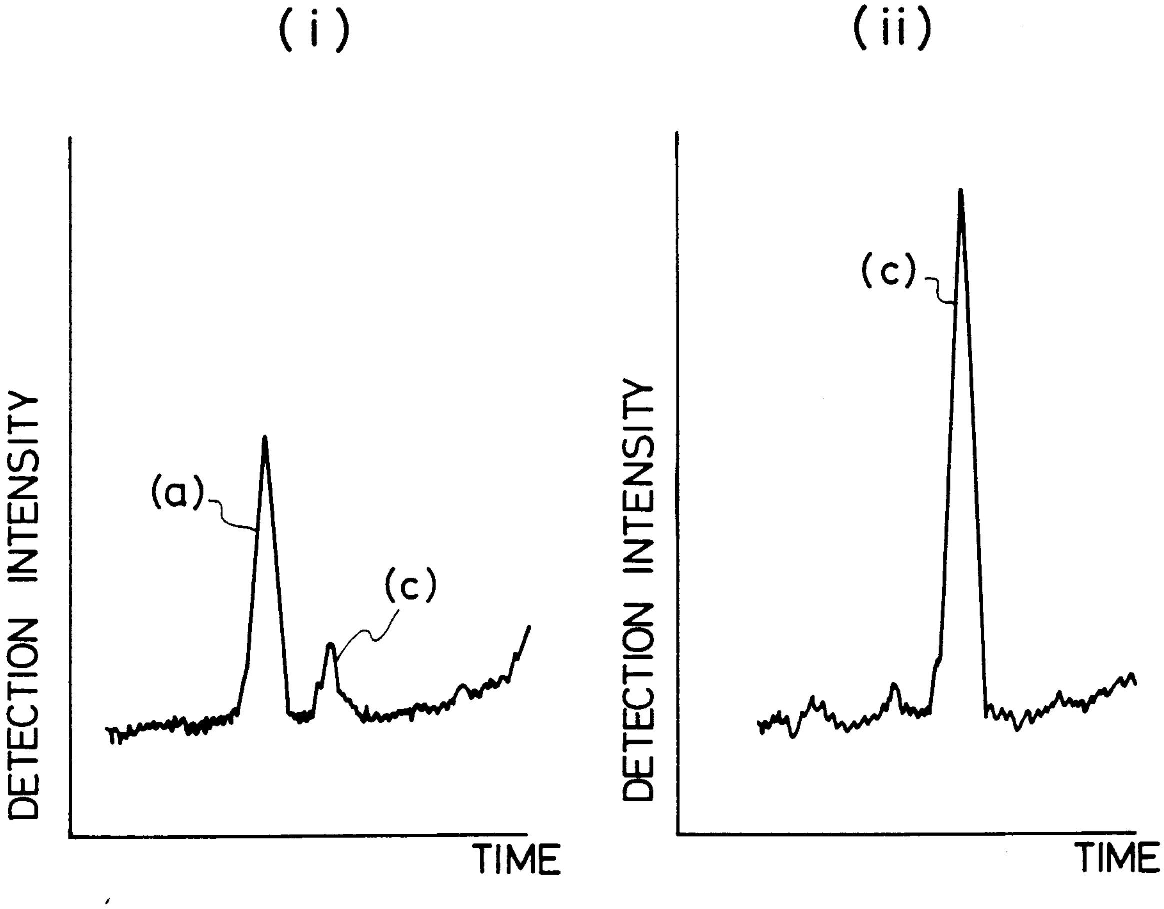
(i)
(ii)
DETECTION INTENSITY
(a)
(c)
TIME
DETECTION INTENSITY
(c)
TIME